(19) Europäisches Patentamt
European Patent Office
Office européen des brevets

(11) Publication number: **0 146 159**
**B1**

(12) **EUROPEAN PATENT SPECIFICATION**

(45) Date of publication of patent specification: **13.01.88**

(51) Int. Cl.$^4$: **C 07 D 295/12**, A 61 K 31/40

(21) Application number: **84201634.7**

(22) Date of filing: **10.11.84**

(54) **Ether of n-propanolamine derivative.**

(30) Priority: **11.11.83 GB 8330197**
**30.11.83 GB 8332023**

(43) Date of publication of application:
**26.06.85 Bulletin 85/26**

(45) Publication of the grant of the patent:
**13.01.88 Bulletin 88/02**

(84) Designated Contracting States:
**AT BE CH DE FR GB IT LI LU NL SE**

(56) References cited:
**WO-A-83/02274**
**DE-A-2 802 864**
**US-A-3 962 238**
**US-E- 30 577**
**ULLMANNS "Encyklopädie der technischen Chemie", ed. 4, vol. 17, "Milchsäure bis Petrolkoks", 1979, VERLAG CHEMIE, Weinheim, pages 453-454**

**The file contains technical information submitted after the application was filed and not included in this specification**

(73) Proprietor: **AKZO N.V.**
**Velperweg 76**
**NL-6824 BM Arnhem (NL)**

(72) Inventor: **Winslow, Eileen**
**21 Loch Laxford**
**East Kilbride Lanarkshire Scotland (GB)**
Inventor: **Basten, Johannes Egbertus Maria**
**Victorstraat 41**
**NL-6654 AV Afferden (GLD.) (NL)**

(74) Representative: **Hermans, Franciscus G.M. et al**
**Postbus 20**
**NL-5340 BH Oss (NL)**

**EP 0 146 159 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel ether of a n-propylamine derivative and acid addition salts thereof, to a method for their preparation and to a pharmaceutical composition containing these compounds as the active principle.

Ethers of n-propanolamine derivatives are known from the U.S. patent specification RE 30.577, where a derivative has been described of the formula:

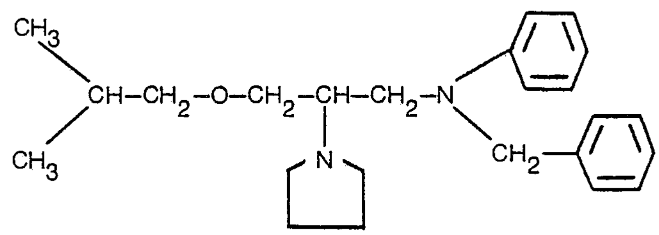

IA

This compound of formula IA, presently known as bepridil, is effective as a medicament in the treatment of cardiovascular disorders. Its clinical use, however, in rare cases been attended by a self limiting rhythm disturbance of the so called ventricular tachycardia type.

It has been noticed that this rhythm disturbance predominantly occurs in elderly patients and often is associated with hypokalaemic conditions of the patient, mainly caused by the use of (non $K^+$-sparing) diuretcis as co-medication.

This rare arrhythogenic potential of the compound many nevertheless cause serious adverse effects in certain patients and may limit the general use of this compound.

The finding of a drug with a better antiarrhythmic activity but without said arrhythmogenic potential would be a definite step forward in the safe treatment of certain high risk patient groups suffering from rhythm disturbances.

Surprisingly it has now been found in relevant animal tests, that both separate optical isomers of the compound of formula I do not possess arrhythmogenic activity under specific hypokalaemic conditions in the rat where the racemate exacerbates arrhythmias.

This finding is the more surprising because it suggests that the adverse arrhythmogenic potential of the racemate is merely caused by the combined presence of the dextro- and levo-rotatory isomers in the racemate.

It has, moreover, been found that the further pharmacological profile of either of the two optical isomers does not differ essentially from that of the racemate. Nevertheless the levo-rotatory isomer does show an increase of anti-arrhythmic activity, as compared with either the racemate of the R-isomer. Its anti-anginal potency is less than that of the racemate and the R-isomer, so that the S-isomer represents a more specific antiarrhythmic compound. Moreover the S-isomer is much more soluble in water than the racemate.

These findings all together render the S-(levo-rotatory)isomer extremely suitable in the treatment of rhythm disturbances especially under circumstances specified above where the racemate is less suitable in view of the arrhythmogenic potential.

The present invention is therefore dealing with the S-(levo-rotatory)isomer of the formula:

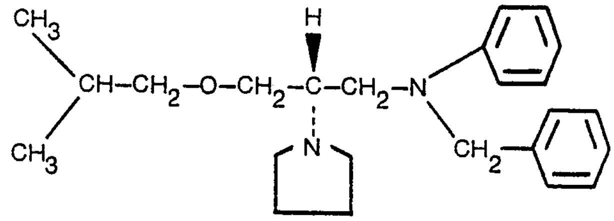

I

and acid addition salts thereof, being substantially free from the corresponding R-isomer, with a process for the preparation of this S-(levo-rotatory)isomer and with pharmaceutical preparations containing this S-(levo-rotatory)isomer as the active constituent and which is essentially free from the corresponding R-isomer.

The S-(levo-rotatory)isomer according to this invention may be prepared by resolution of the racemate using well known methods for resolving racemates. It was found, however, that — by using these conventional methods — the yields were extremely low, so that these methods are not suitable for large scale production.

A very suitable method for preparing the S-(levo-rotatory)isomer according to the invention is starting with the resolution of a racemic compound of the formula II

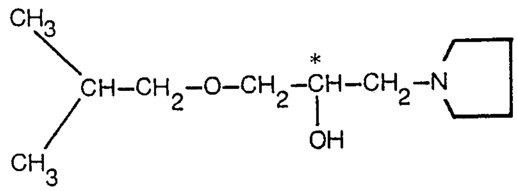 II

(the asterisk indicates the chiral carbon atom). This resolution can be carried out in a conventional manner using well known optically active carboxylic acids. The use of L-(−)dibenzoyl tartaric acid is preferred.

One of the optically active isomers of formula II thus obtained is subsequently converted into the S-(levo-rotatory)isomer of formula I using the following reaction steps:

(1) 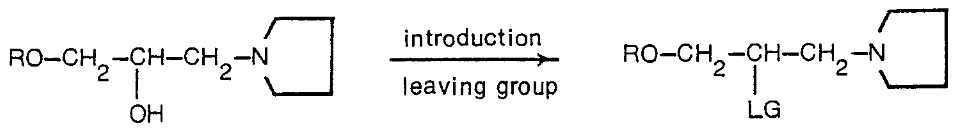


R–isomer R–isomer

or S–isomer

(2) 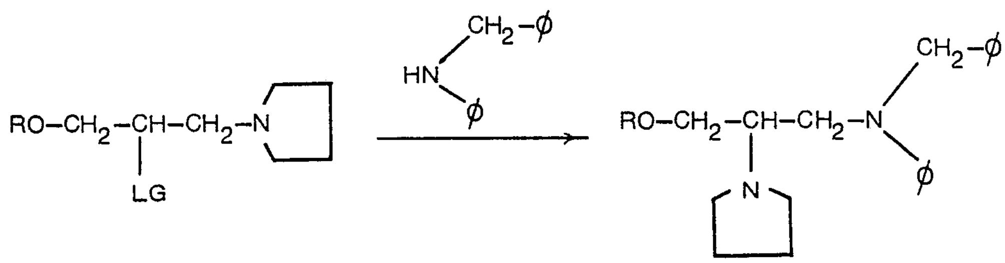

R–isomer S–isomer

LG = leaving group such as halogen.

R = isobutyl 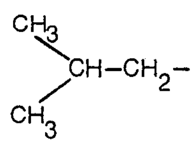

ϕ = phenyl

The leaving group can be introduced with retention of configuration or through a $SN_2$ type reaction mechanism causing inversion, depending on the method selected. Using, for example, $SOCl_2$ in toluene as LG-introducing agent the levo-rotatory alcohol of formula II is converted into its dextro-rotatory halogen derivative (R-isomer).

Pharmaceutically acceptable acid addition salts of the S-(levo-rotatory)isomer according to this invention are for example derived from hydrochloric acid, hydrobromic acid, sulphuric acid, methanesulphonic acid, acetic acid, maleic acid, fumaric acid, tartaric acid acid, ascorbic acid and citric acid. Depending upon the water content of the final reaction mixture, the S-(levo-rotatory)isomer or its acid addition salt, isolated therefrom, may further be obtained in a hydrated form.

As already said the use of the S-(levo-rotatory)isomer according to this invention (and acid addition salts thereof) being substantially free from the corresponding R-isomer is extremely advantageous in the treatment of rhythm disturbances but especially in those circumstances where rhythm disturbances are accompanied with abnormally low plasma potassium levels (hypokalemic conditions), which for example may be caused by the use of diuretics as co-medication.

The S-(levo-rotatory)isomer according to formula I and acid addition salts thereof may be administered enterally or parenterally, whereby the administration per os and through a continuous infusion into the vein is to be preferred.

Mixed with suitable carriers the compounds of the invention can be processed into a form which is suitable for enteral administration such as pills, tablets, capsules and suppositories. For injection or infusion purposes, the compounds of the present invention are dissolved, emulsified or suspended in a suitable liquid.

The S-(levo-rotatory)isomer according to this invention is preferably administered in a daily dosage of 0.5—20 mg per kg bodyweight. For oral administration to human beings the preferred daily dosage varies from 50—500 mg and for parenteral administration from 25—1000 mg.

## Examples

1. S-α[(2-methylpropoxy)methyl]-1-pyrrolidine ethanol

250.0 g Of racemic α[(2-methylpropoxy)-methyl]-1-pyrrolidine-ethanol were dissolved in 1 liter anhydrous ethanol after which 467 g (−) dibenzoyl tartaric acid monohydrate was added to the solution.

The precipitated salt crystals, obtained by filtration were then dried and recrystallised 4 times from anhydrous ethanol. Melting point of the S-salt = 143.2—146.7°C and $[\alpha]_D^{20} = -92.9°$ (c = 0.5 $CH_3OH$).

This levo-rotatory salt was added to 758 ml of 1 n NaOH, after which the suspension was extracted with ether (3×) and the combined ether extracts washed with 120 ml water (5×). The ether extracts were then dried over magnesium sulphate whereupon the ether solvent was evaporated. The residue was distilled in vacuo.

Yield of the free base: 45.6 g (36%);
boiling point (0.3 mm): 83—90°C;
$[\alpha]_D^{20}$: −13.06° (c = 0.55 $CH_3OH$).

2. R-1-[2-chloro-3-(2-methylpropoxy)-propyl]pyrrolidine

45.6 g Of the levo-rotatory isomer obtained in 1. were dissolved in 135 ml anhydrous toluene and the solution was heated up to 40—45°C.

Subsequently a solution of 35 ml thionylchloride in 45 ml toluene was slowly added to the previous solution in about 4 hours while stirring. The reaction mixture was heated to 70—75°C and stirred for 2 hours. The mixture was then cooled down to 0°C whereupon water (about 45 ml) was added very slowly (the temperature was not allowed to rise above 20°C.

The mixture was then poured into ice-water, after which the toluene layer was separated and extracted with water again. To the combined water layers 135 ml toluene were added whereupon the two-layers system was adjusted to pH 9 by adding concentrated ammonia.

The two layers were separated. The toluene layer thus obtained was washed with water dried over magnesium sulphate and evaporated.

Yield: 44.4 g oily substance (89%);
$[\alpha]_D^{20} = +24.66°$ (c = 2; $CH_3OH$);
$R_f = 0.42$ in methylene chloride : methanol (5:1) on $SiO_2$.

3. S-β-[(2-methylpropoxy) methyl]-N-phenyl-N-(phenylmethyl)-1-pyyrolidine-ethanamine.HCl $H_2O$

A solution of 40.58 g N-benzylaniline in toluene was heated to about 80°C and then slowly added (in about 30 minutes) to 10.0 g sodiumamide, suspended in 100 ml anhydrous toluene, while stirring and under nitrogen atmosphere. THe mixture was refluxed for two hours and cooled down to about 70°C. Subsequently a solution of 44.4 g of the R-(dextro-rotatory)isomer (obtained in 2.) in 45 ml toluene was slowly added to the reaction mixture in about ½ hour.

The mixture was refluxed for 2 hours; then it was cooled down to about 10°C, whereupon 45 ml water was slowly added.

The mixture obtained was poured into 180 ml ice-water after which it was stirred for 15 minutes.

The toluene layer was separated from the water layer, after which the toluene layer was washed, dried and evaporated, yielding about 82 grams oily substance.

The oil was purified by chromatography over a $SiO_2$ column and subsequently over a $Al_2O_3$ column. Yielding about 54 g of an oily substance.

The purified oil was then dissolved in about 2000 ml ether. To this solution 75 ml 3.7 n HCl in ethanol was slowly added, after which the solvents were evaporated. The oily residue was subsequently dissolved in 500 ml moist ethylacetate. After cooling down this solution to about −50°C crystals of the hydrochloride salt were obtained.

Yield: 27.5 g of the HCl salt (containing 1 mol. $H_2O$), melting point: 73—75.5°C;
$[\alpha]_D^{20} = -14.90°$ (c = 1; $CH_2Cl_2$).
$R_f$ in n-butanol : acetic acid : water (4:1:5) = 0.65 on $SiO_2$.

**Claims**

1. The S-(levo-rotatory)isomer of the formula:

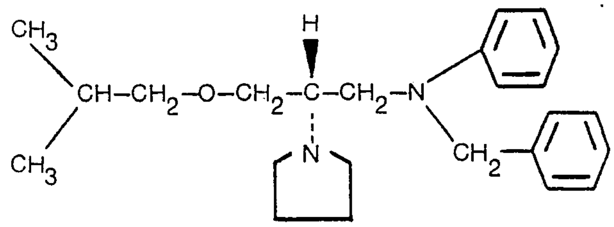

I

and pharmaceutically acceptable acid addition salts thereof, being essentially free from the corresponding R-(dextro-rotatory)isomer.

2. A pharmaceutical preparation containing as the active principle the levo-rotatory isomer described in claim 1 or a phramaceutically acceptable acid addition salt thereof, which preparation is essentially free from the corresponding R-(dextro-rotatory)isomer.

**Patentansprüche**

1. Das (links-drehende) S-Isomer der Formel:

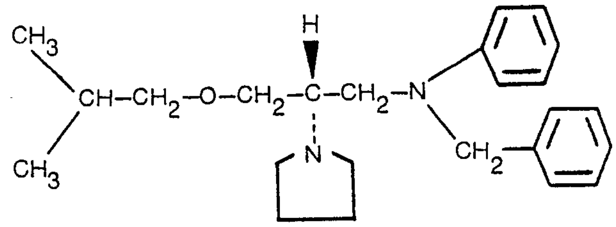

I

und dessen pharmazeutisch unbedenkliche Salze, im wesentlichen frei vom entsprechenden R-Isomer.

2. Pharmazeutisches Präparat dass als Wirkstoff das S-Isomer nach Anspruch 1 oder ein pharmazeutisch unbendenkliches Salz davon enthält, welches Präparat im wesentlichen frei ist vom entsprechenden R-Isomer.

**Revendications**

1. L'isomère S (lévogyre) de formule:

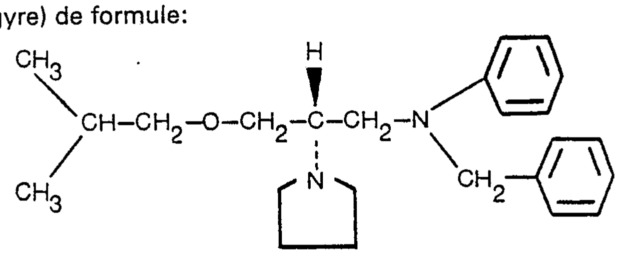

I

et ses sels d'addition d'acide pharmaceutiquement acceptables, essentiellement exempts de l'isomère R (dextrogyre) correspondant.

2. Une préparation pharmaceutique contenant, comme principe actif, l'isomère lévogyre décrit dans la revendication 1 ou un de ses sels d'addition d'acides pharmaceutiquement acceptables, laquelle préparation est essentiellement exempte de l'isomère R (dextrogyre) correspondant.